# EUROPEAN PATENT APPLICATION

(11) **EP 2 692 417 A1**
(43) Date of publication of application: **05.02.2014**
(21) Application number: 12178728.7
(22) Date of filing: 31.07.2012
(51) Int. Cl.: B01D 61/58, C02F 1/66, C02F 1/44, B01D 61/00, C05F 5/00, C05F 9/00, C05F 3/00, C05F 7/00, C05F 17/00, C02F 11/04

(54) **Organic forward osmosis system**

(71) Applicant: Hydration Systems, Llc, Scottsdale, AZ 85258 (US)
(72) Inventor: Herron, John R, Corvallis, OR 97333 (US); Lampi, Keith, Corvallis, OR 97333 (US); Gormly, Sherwin, Carson City, NV 89703 (US); Beaudry, Edward, Corvallis, OR 97330 (US)
(74) Representative: Potter Clarkson LLP

(57) **Abstract**

An organic forward osmosis system (1) includes an acid treatment stage comprising an acid treatment operation (40) configured to produce an acid treated, digester centrate stream in a forward osmosis reject loop (70) and a fertilizer stream. A forward osmosis stage is coupled to the acid treatment stage and includes a forward osmosis operation (74) configured to remove water from the acid treated, digester centrate stream by: diverting the acid treated, digester centrate stream to one side of at least one forward osmosis membrane; and contacting an opposite side of the at least one forward osmosis membrane with a salt brine stream in a forward osmosis draw loop and osmotically pulling water across the at least one forward osmosis membrane from the acid treated, digester centrate stream to the salt brine stream using only a concentration gradient; thereby producing a concentrated, acid treated, digester centrate stream and a diluted salt brine stream.

## Description

### BACKGROUND

### Technical Field

This document relates to organic forward osmosis systems and processes that provide for the combined and simultaneous treatment of centrate wastewater from anaerobic digestion, reverse osmosis membrane water treatment waste brines, and the production of high-grade organic fertilizer.

### Background

The wastewater or centrate that is drawn from the bottom of anaerobic agricultural waste digester tanks is high in dissolved and colloidal solids. These anaerobic digesters are primarily intended for the producing of methane gas and dry composted solids. The centrate that is drawn out of these digesters are generally much higher in nitrogen than phosphorus (not a valuable balanced fertilizer). At the same time this centrate is so high in ultra fine materials that will blind and/or foul most known filtration equipment. Even after pretreatment precipitation these fine solids would foul most membrane and filtration processes.

### SUMMARY

Aspects of this document relate to organic forward osmosis systems and processes. These aspects may include, and implementations may include, one or more or all of the components and steps set forth in the appended CLAIMS, which are hereby incorporated by reference.

In one aspect, an organic forward osmosis system for concentrating digester biomass intended for Organic Certified fertilizer is disclosed. The system may include an acid treatment stage comprising an acid treatment operation configured to produce an acid treated, digester centrate stream in a forward osmosis reject loop and a fertilizer stream. A forward osmosis stage may be coupled to the acid treatment stage and configured to receive the acid treated, digester centrate stream from the acid treatment stage. The forward osmosis stage may include a forward osmosis operation configured to remove water from the acid treated, digester centrate stream by: diverting the acid treated, digester centrate stream to one side of at least one forward osmosis membrane; and contacting an opposite side of the at least one forward osmosis membrane with a salt brine stream in a forward osmosis draw loop and osmotically pulling water across the at least one forward osmosis membrane from the acid treated, digester centrate stream to the salt brine stream using only a concentration gradient; and thereby producing a concentrated, acid treated, digester centrate stream and a diluted salt brine stream.

Particular implementations may include one or more or all of the following.

The diluted salt brine stream may be maintained in the forward osmosis draw loop.

The system may also be for recovering water for re-use and may include a reverse osmosis stage coupled to the forward osmosis stage configured to receive the diluted salt brine stream from the forward osmosis stage. The reverse osmosis stage may include a reverse osmosis operation configured to pump under pressure the diluted salt brine stream to at least one reverse osmosis membrane and produce a re-concentrated salt brine stream maintained in the forward osmosis draw loop and a recycled purified water stream for re-use.

The acid treatment stage may be configured to augment digester biomass with phosphorus and the acid treatment operation comprises using Organic Certified phosphoric acid at an Organic Certifiable process pH of 3.5, thereby balancing a P:K:N ratio of the fertilizer stream. Likewise the N level may be raised by using nitric acid for part of the acidification. Acidification could also be effected with pressurized CO2 addition.

The salt brine stream may include an Organic Certified compatible potash salt. The potash salt may include one of potassium chloride, potassium carbonate, potassium sulfate, potassium magnesium sulfate, langbeinite, and potassium nitrate.

The at least one forward osmosis membrane may be a semipermeable thin film composite membrane in one of a fiber, plate and frame, and spiral wound membrane configuration, a semipermeable cellulosic membrane in one of a fiber, plate and frame, and spiral wound membrane configuration, or any combination thereof.

In another aspect, an organic forward osmosis system that simultaneously treats digester centrate, recovers digester biomass intended for Organic Certified fertilizer, and augments that biomass with phosphorus, nitrate or CO2 is disclosed. The system may include a digester stage including a digesting operation configured to produce a digester centrate stream from waste. An acid treatment stage may be coupled to the digester stage and configured to receive the digester centrate stream and augment digester biomass with phosphorus or nitrate or CO2. The acid treatment stage may include an acid treatment operation that uses Organic Certified phosphoric acid, nitric acid or CO2 at an Organic Certifiable process pH of 3.5 to produce an acid treated, digester centrate stream in a forward osmosis reject loop and a fertilizer stream with a balanced P:K:N ratio. A forward osmosis stage may be coupled to the acid treatment stage and configured to receive the acid treated, digester centrate stream from the acid treatment stage. The forward osmosis stage may include a forward osmosis operation configured to remove water from the acid treated, digester centrate stream by: diverting the acid treated, digester centrate stream to one side of at least one forward osmosis membrane; and contacting an opposite side of the at least one forward osmosis membrane with a salt brine stream comprising an Organic Certified compatible potash salt in a forward osmosis draw loop and osmotically pulling water across the at least one forward osmosis membrane from the acid treated, digester centrate stream to the salt brine stream using only a concentration gradient; and thereby producing a concentrated, acid treated, digester centrate stream and a diluted salt brine stream maintained in the forward osmosis draw loop.

Particular implementations may include one or more or all of the following.

The system may also be for recovering water for re-use and may include a reverse osmosis stage coupled to the forward osmosis stage configured to receive the diluted salt brine stream from the forward osmosis stage. The reverse osmosis stage may include a reverse osmosis operation configured to pump under pressure the diluted salt brine stream to at least one reverse osmosis membrane and produce a re-concentrated salt brine stream maintained in the forward osmosis draw loop and a recycled purified water stream for re-use.

The salt brine stream may include an Organic Certified compatible potash salt. The potash salt may include one of potassium chloride, potassium carbonate, potassium sulfate, potassium magnesium sulfate, langbeinite, and potassium nitrate.

The system may also include a screening stage coupled to the digester stage and configured to receive the digester centrate stream. The screening stage may include a screening operation configured to produce a screened, digester centrate stream and a screened reject stream. The acid treatment stage may be coupled to the screening stage and configured to receive the screened, digester centrate stream. A filtering stage may be coupled to the acid treatment stage and configured to receive the fertilizer stream and may include a filtering operation configured to produce a filtered, fertilizer stream. A drying stage may be coupled to both the screening stage and the filtering stage configured to receive the screened reject stream and the filtered, fertilizer stream, the drying stage and may include a drying operation configured to produce fertilizer.

The at least one forward osmosis membrane may be a semipermeable thin film composite membrane in one of a fiber, plate and frame, and spiral wound membrane configuration, a semipermeable cellulosic membrane in one of a fiber, plate and frame, and spiral wound membrane configuration, or any combination thereof.

In still another aspect, an organic forward osmosis system that simultaneously treats digester centrate, recovers digester biomass intended for Organic Certified fertilizer, augments that biomass with phosphorus or nitrogen or CO2, and recovers water for re-use is disclosed. The system may include a digester stage including a digesting operation configured to produce a digester centrate stream from waste. An acid treatment stage may be coupled to the digester stage and configared to receive the digester centrate stream and augment digester biomass with phosphorus or nitrogen or CO2. The acid treatment stage may include an acid treatment operation that uses Organic Certified phosphoric acid, nitric acid, or CO2 at an Organic Certifiable process pH of 3.5 to produce an acid treated, digester centrate stream in a forward osmosis reject loop and a fertilizer stream with a balanced P:K:N ratio. A forward osmosis stage may be coupled to the acid treatment stage and configured to receive the acid treated, digester centrate stream from the acid treatment stage. The forward osmosis stage may include a forward osmosis operation configured to remove water from the acid treated, digester centrate stream by: diverting the acid treated, digester centrate stream to one side of at least one forward osmosis membrane; and contacting an opposite side of the at least one forward osmosis membrane with a salt brine stream comprising an Organic Certified compatible potash salt in a forward osmosis draw loop and osmotically pulling water across the at least one forward osmosis membrane from the acid treated, digester centrate stream to the salt brine stream using only a concentration gradient; and thereby producing a concentrated, acid treated, digester centrate stream and a diluted salt brine stream maintained in the forward osmosis draw loop. A reverse osmosis stage may be coupled to the forward osmosis stage configured to receive the diluted salt brine stream from the forward osmosis stage. The reverse osmosis stage may include a reverse osmosis operation configured to pump under pressure the diluted salt brine stream to at least one reverse osmosis membrane and produce a re-concentrated salt brine stream maintained in the forward osmosis draw loop and a recycled purified water stream for re-use.

Particular implementations may include one or more or all of the following.

The system may also be for recovering water for re-use and may include a reverse osmosis stage coupled to the forward osmosis stage configured to receive the diluted salt brine stream from the forward osmosis stage. The reverse osmosis stage may include a reverse osmosis operation configured to pump under pressure the diluted salt brine stream to at least one reverse osmosis membrane and produce a re-concentrated salt brine stream maintained in the forward osmosis draw loop and a recycled purified water stream for re-use.

The salt brine stream may include an Organic Certified compatible potash salt. The potash salt may include one of potassium chloride, potassium carbonate, potassium sulfate, potassium magnesium sulfate, langbeinite, and potassium nitrate.

The system may also include a screening stage coupled to the digester stage and configured to receive the digester centrate stream. The screening stage may include a screening operation configured to produce a screened, digester centrate stream and a screened reject stream. The acid treatment stage may be coupled to the screening stage and configured to receive the screened, digester centrate stream. A filtering stage may be coupled to the acid treatment stage and configured to receive the fertilizer stream and may include a filtering operation configured to produce a filtered, fertilizer stream. A drying stage may be coupled to both the screening stage and the filtering stage configured to receive the screened reject stream and the filtered, fertilizer stream, the drying stage and may include a drying operation configured to produce fertilizer.

The at least one forward osmosis membrane may be a semipermeable thin film composite membrane in one of a fiber, plate and frame, and spiral wound membrane configuration, a semipermeable cellulosic membrane in one of a fiber, plate and frame, and spiral wound membrane configuration, or any combination thereof.

According to a further aspect there is provided an apparatus comprising:
a separation tank configured to subject a biomass digester centrate to an acidic treatment to produce (i) an acid-treated centrate stream and (ii) a stream of wet solids; and
a forward osmosis loop configured to subject the acid-treated centrate stream to forward osmosis to produce (i) a concentrated acid-treated centrate stream and (ii) a diluted forward osmosis osmotic agent solution stream.

The apparatus may further comprise a digester system configured to digest the centrate prior to providing the centrate to the separation tank.

The apparatus may further comprise a screen configured to remove solid particles from the centrate prior to providing the centrate to the separation tank.

The forward osmosis loop may comprise a forward osmosis membrane. One side of the forward osmosis membrane may be configured to be exposed to the acid-treated centrate stream. An opposite side of the forward osmosis membrane may be configured to be exposed to a forward osmosis agent solution in a forward osmosis draw loop. In use, water may be osmotically pulled across the forward osmosis membrane from the acid-treated digester centrate stream to the osmosis agent solution using only a concentration gradient. This may produce (i) the concentrated, acid-treated centrate stream and (ii) the diluted forward osmosis osmotic agent solution stream.

The apparatus may further comprise an air drying box configured to receive the stream of wet solids produced by the separation tank.

The apparatus may further comprise a filter configured to filter, and optionally dry, the stream of wet solids and provide the filtered stream of wet solids to the air drying box.

The apparatus may further comprise a reverse osmosis system. The reverse osmosis system may be configured to subject the diluted forward osmosis osmotic agent solution stream to reverse osmosis, to produce (i) a concentrated osmotic agent solution stream and (ii) a substantially pure water stream.

The apparatus may be configured to use the concentrated osmotic agent solution stream produced by the reverse osmosis system in the forward osmosis loop.

The apparatus may further comprise a filter configured to filter the stream of wet solids to produce a reject particle stream and a screened liquid stream.

The apparatus may further comprise an air drying box configured to dry the reject particle stream. The apparatus may further comprise a filter configured to subject the screened liquid stream to ultrafiltration or microfiltration, to produce a stream of produced water and stream of reject water.

The apparatus may be configured to return the stream of produced water to the forward osmosis loop.

The apparatus may be configured to combine the reject water stream with the centrate.

The foregoing and other aspects, features, and advantages will be apparent to those of ordinary skill in the art from the DESCRIPTION and DRAWINGS, and from the CLAIMS.

### BRIEF DESCRIPTION OF DRAWINGS

Implementations will hereinafter be described in conjunction with the appended DRAWINGS (which are not necessarily to scale), where like designations denote like elements.

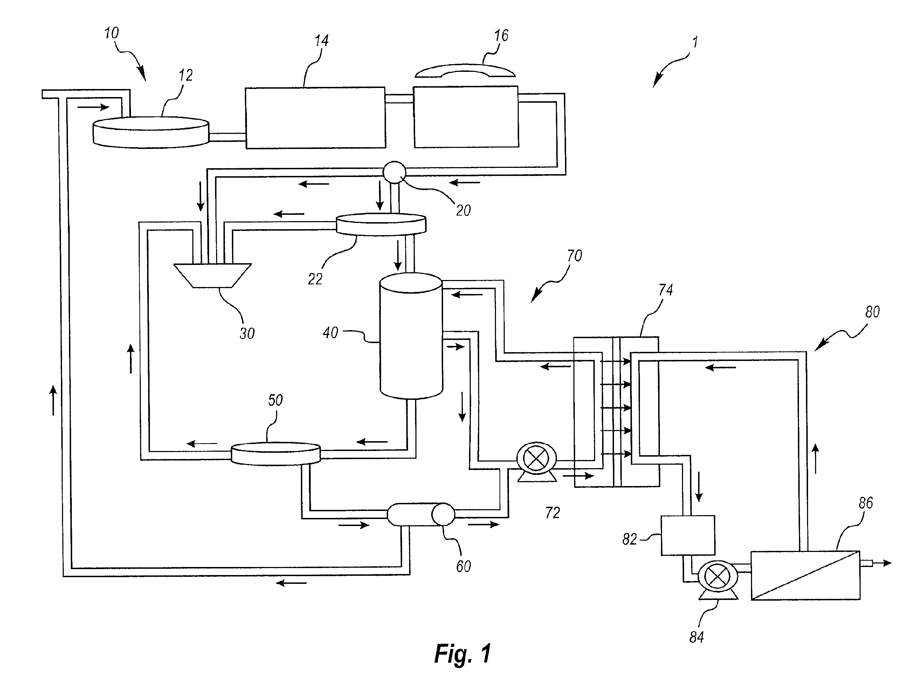

FIG. 1 is a schematic block diagram of an implementation of an organic forward osmosis system.

### DESCRIPTION

This document features organic forward osmosis systems. These systems embody the use of an acidic pretreatment of anaerobic digester centrate by the addition of phosphoric acid, nitric acid or CO2 with a follow-on use of forward osmosis (FO) with reverse osmosis (RO) for the simultaneous production of organic fertilizer and advanced water treatment. The systems and processes harvest pretreatment process sludge as fertilizer with the phosphorous and nitrogen integrated into it. This integration of processes into a single system (the combination of a phosphorus and nitrogen adding centrate pretreatment with FO/RO water production) achieves the nutrient (nitrogen to phosphorous) balancing of fertilizer production from agricultural biomass digesters while simultaneously harvesting potentially potable water grade water from the process.

The use of phosphoric acid used in organic fertilizer production to separate out the bulk of the useful organic and nitrogen material prior to the membrane based recovery of 50% to 80% of the digester centrate water for high grade water recycle for processes normally requiring new fresh water at or near drinking water quality is a unique synergy that has not been previously achieved. By integrating the water recovery with the required additions for balancing the fertilizer the water recovery chemical treatment cost and resource use is essentially 100% offset by the production requirements of fertilizer production.

There are many features of organic forward osmosis system implementations disclosed herein, of which one, a plurality, or all features or steps may be used in any particular implementation. In the following description, reference is made to the accompanying DRAWINGS which form a part hereof, and which show by way of illustration possible implementations. It is to be understood that other implementations may be utilized, and structural, as well as procedural, changes may be made without departing from the scope of this document. As a matter of convenience, various components will be described using exemplary materials, sizes, shapes, dimensions, and the like. However, this document is not limited to the stated examples and other configurations are possible and within the teachings of the present disclosure.

### Overview

FO provides a solution to the thermodynamics for a low value high mass application like fertilizer production. FO provides a non-membrane fouling anaerobic digester (e.g. anaerobic bacteria) biomass harvest and fertilizer production in combination with both phosphorus and nitrogen nutrient balancing and water treatment and high quality water recovery.

FO is a membrane technology that uses membranes with similar selectivity to those used in RO. But instead of applying high pressure to squeeze water from a solution, FO uses a solution with high osmotic potential to draw water through the membrane from a solution of low osmotic potential. The FO process (also termed direct osmotic concentration) has been described in an earlier patent (Herron et al. US 5,821,430), which is hereby incorporated by reference.

FO was developed as a membrane based food processing water removal process, and thus can simultaneously remove water from liquid slurries similar to concentrated anaerobic digester centrate and produce RO level treated water as a byproduct. It should be noted that the recovery of nutrients at the FO membrane has been used to retain subtle flavors and color compounds of food products and will be important to the organic fertilizer production and exquisitely thorough recovery of complex organic nutrients for use in the fertilizer. FO/RO will also deny these same nutrients to any receiving waters where these same nutrients would promote growth of aquatic plants and algae in the environment where that growth is not desirable.

Far less energy is required to separate and dewater digesters using an FO membrane than other separation processes. If a constant source of brine is available that brine will provide better than 90% water removal (the recovery of up to 90% of the centrate water after acidic precipitation) and the retention of up to 98% of the nutrient value of the centrate water that is removed from the centrate by precipitation (the supernate) with no further energy input to the process than that required to bring both the digester bearing wastewater to one side of the membrane and the osmotic agent or brine to the other side of the membrane.

FO/RO will allow for high-grade water reuse production from high ammonia nitrogen (but low phosphorus) agricultural digester centrate/wastewater. This synergistic process is uniquely valuable to organic food production and processing operations, and represents a significant potential advance in sustainable food process technology.

### Systems and Processes

System implementations combine a phosphorus and nitrate adding centrate pretreatment with FO/RO water production that achieves the nutrient (nitrogen to phosphorous) balancing of fertilizer production from agricultural biomass digesters while simultaneously harvesting potentially potable water grade water.

There are a variety of organic forward osmosis system implementations that simultaneously treat digester centrate, recover digester biomass, augment that biomass with phosphorus, and recover water for reuse. Notwithstanding, turning to FIG. 1 and for the exemplary purposes of this disclosure, organic forward osmosis system 1 and its related process is shown. In system 1, FO is used in conjunction with RO to concentrate the waste stream and achieve high quality water recovery.

First, in a digester stage, bacteria or other digesters digest the food waste or other appropriate waste. For example, primary settling and one or more stages of anaerobic digestion may occur first in a standard anaerobic digester system 10 having a settling tank 12, a tank with water baffles 14 for example followed by a tank with a variable surface lid 16 for example to account for the heat and gases (e.g. methane, carbon dioxide, etc.) from the digesters. The released methane gas can be collected and burned in a modified diesel generator for example to produce electricity.

Next, in a screening stage, the clarified residual liquid (the digester centrate) is then decanted off and pumped through a rough screening operation (here represented by an auger screen 20 for example). 200 to 400 micron screening is done at this primary screen stage and is a common practice for recovery of larger particles of plant and animal waste commonly entrained in the liquid centrate drained for food processing related digesters. Shaker screen 22 is then used to remove solids particles down to about 100 microns. In a drying stage, the resulting larger particles that were screened (the reject) are diverted to an air drying bin 30, while the screened digester centrate is diverted to the primary separation tank 40.

At this point the suspended solids remaining in the screened digester centrate stream are colloidal or very slow settling but also quite problematic for all common forms of filtration or diffusion based membrane separation. This material is amenable to FO membrane separation but is present at a concentration that would greatly overload this process too quickly to maintain operations. The process for recovery of usable water from this screened digester centrate stream is enabled in the separation tank 40 by the use of acidification and precipitation by pH adjustment from the ammonia driven high pH values of the digester liquids (over pH 8) to well below the CO2 equivalency point (generally at or above 3.5). This acidification stage causes a precipitation driven separation of solids referred to as sweep-floe.

By using organically certifiable phosphoric acid and nitric acid (and possibly other balancing organically certified acid products (e.g., citric acid, sulfuric acid, and the like)) and having end point precipitation tank pHs near 3.5, the acidification process remains organically certifiable as well as an effective way to add necessary phosphors, nitrates and other components (e.g. sulfurs) to the wet solids at the bottom of the precipitation tank 40. In this way the common task of acidification pretreatment of the centrate wastewater is combined with the task of harvesting and nutrient balancing the solids that are to be re-tasked as marketable fertilizer. This will result in the correct balance of phosphors and other components (e.g. sulfurs) in the system and achieve the final fertilizer needs and N:P:K(:S) ratios.

Turning back to the process flow, in a filter stage wet solids at the bottom of the precipitation tank 40 are pumped through a sludge filter, belt press, screen, or any other filtration system 50. 15 to 60 micron screening is done at this stage. The resulting larger particles that were filtered (the reject) are diverted to the air drying bin 30 to be re-tasked as marketable fertilizer, while the screened liquid is drawn into an ultra-filtration (UF) or Microfiltration (MF) membrane contactor 60. The UF reject water is returned to the anaerobic digester system 10 for enhanced concentration while the UF produced water is drawn into the FO loop 70 of the FO stage (described next).

The FO stage is a process that involves selective mass transfer across a membrane that allows a desired component to cross the membrane from a solution of higher concentration of the component to a solution of lower concentration. A semi-permeable membrane allows water to pass but blocks the movement of dissolved species.

The membrane may have a design similar to that disclosed in U.S. Patent No. 4,033,878 to Foreman et al., entitled "Spiral Wound Membrane Module for Direct Osmosis Separations," issued July 5, 1977, the disclosure of which is hereby incorporated entirely herein by reference. A spiral wound membrane design configuration is inexpensive and can provide one of the greatest membrane surface areas in a vessel per cost (it can have a high membrane density (about 30 m² per 20 cm diameter by 100 cm long element)).

In general, a spiral wound configuration, a permeate spacer, a feed spacer and two membranes can be wrapped around a perforated tube and glued in place. The membranes are wound between the feed spacer and the permeate spacer. Feed fluid is forced to flow longitudinally through the module through the feed spacer, and fluid passing through the membranes flows inward in a spiral through the permeate spacer to the center tube. To prevent feed fluid from entering the permeate spacer, the two membranes are glued to each other along their edges with the permeate spacer captured between them. The feed spacer remains unglued. Module assemblies are wound up to a desired diameter and the outsides are sealed.

In use, the membrane forces a draw solution (i.e., brine) to flow through the entire, single membrane envelope. The brine is pumped into one end of a center tube with perforations. A barrier element fixed halfway down the tube forces the brine flow through the perforations into the membrane envelope. A glue barrier is applied to the center of the membrane envelope so that fluid must flow to the far end of the membrane where a gap allows it to cross over to the other side of the membrane envelope then back into the second half of the center tube and out of the element. While a single envelope can be employed, there may be multiple envelopes wound/wrapped around the center tube with feed fluid spacers between the envelopes. Furthermore, a plurality of membranes may be used and may operate in a parallel flow configuration.

Here in FIG. 1, because the driving force causing the transfer of mass through the FO membrane is osmotic pressure, no additional energy input is required to cause the transfer to occur beyond what is required to place the solutions in contact with the membrane (through transfer pump 72, etc.). Water moves from the waste to the brine due to a concentration gradient and not due to applied pressure or heat or any other power input.

As a result, as salt brine is contacted to one side of the FO membrane and dilute wastewater is contacted to the opposite side, water will diffuse through the membrane from the wastewater to the brine. The semi-permeable membrane will keep unwanted impurities and sediment in the wastewater, thus, producing clean diluted brine. Depending upon the material used for the membrane, the structure of the membrane, and the arrangement of the membrane within the system, the amount and rate of transfer may be enhanced and/or controlled.

Specifically, at the point of acidification, the majority of the solids have been removed, but the supernatant in the primary separation tank 40 still contains a great deal of potentially high fouling materials that are fine enough to pass most filters but large enough to cause fouling of most membrane processes. FO provides membrane rejection and recovery of these partials and ionic contaminates while allowing over 90% of the water to be recovered and then produced by the downstream RO system 86, which re-concentrates the osmotic agent (OA) to drive the FO. The OA may be composed of an Organic certification compatible potash salt (e.g. potassium chloride, potassium carbonate, potassium sulfate, potassium magnesium sulfate, langbeinite, potassium nitrate, and the like). The FO/RO combination provides an extremely low fouling membrane water treatment process for wastewaters like the pretreated supernatant. This combination of FO/RO with the phosphoric-nitric pretreatment and the synergistic nutrient balancing effect combine to produce the novel process.

Thus, during the FO stage the supernatant and UF produced water (high grade water consisting of 50% to 80% recovered digester centrate water) is then flowed through the FO membrane element 74 by transfer pump 72 and recycled. The OA or brine (high osmotic potential draw solution) from RO reject is pumped through the other side of the FO element 74 (i.e. on the other side of the FO membrane within the element 74). Water drawn across the membrane due to osmotic pull will dilute the OA or brine, which is then returned to the OA tank 82. The OA or brine is re-concentrated and maintained in a FO draw solution loop 80. FO operates at low pressures thereby reducing fouling of the membrane. The only pressures applied are to provide circulation and are typically 1 to 2 Bar.

For the recovery of water, FO is followed by the collection of the diluted OA or brine into the OA tank 82. Re-concentration of the brine after it absorbs water from the supernatant and UF produced water stream is accomplished by RO. Diluted OA or brine from the OA tank is pumped by transfer pump 84 under pressure to the RO system 86 elements where it is re-concentrated to appropriate draw strength, and the clean product water is simultaneously produced. This completes the recovery of wastewater to high-grade reuse water (e.g., 95% of the value of the wastewater). Thus, the RO system 86 produces a purified water stream that can be reused.

Thus, the wastewater can be from a food waste methane digester and FO can convert it into a useful fertilizer. Potassium Chloride brine can be used as the osmotic agent so that the fertilizer can be certified as Organic. The diluted brine can be re-concentrated by RO, delivering a purified water stream that can be reused in the food processing plant for example.

### Specifications, Materials, Manufacture, Assembly

It will be understood that implementations are not limited to the specific components disclosed herein, as virtually any components consistent with the intended operation of an organic forward osmosis system may be utilized. Accordingly, for example, although particular components and so forth, are disclosed, such components may comprise any shape, size, style, type, model, version, class, grade, measurement, concentration, material, weight, quantity, and/or the like consistent with the intended operation of a organic forward osmosis system implementation. Implementations are not limited to uses of any specific components, provided that the components selected are consistent with the intended operation of an organic forward osmosis system implementation.

Accordingly, the components defining any organic forward osmosis system implementation may be formed of any of many different types of materials or combinations thereof that can readily be formed into shaped objects provided that the components selected are consistent with the intended operation of a organic forward osmosis system implementation. For example, the components may be formed of: rubbers (synthetic and/or natural) and/or other like materials; glasses (such as fiberglass), carbon-fiber, aramid-fiber, any combination thereof, and/or other like materials; polymers such as thermoplastics (such as ABS, Acrylic, Fluoropolymers, Polyacetal, Polyamide; Polycarbonate, Polyethylene, Polysulfone, and/or the like), thermosets (such as Epoxy, Phenolic Resin, Polyimide, Polyurethane, Silicone, and/or the like), any combination thereof, and/or other like materials; composites and/or other like materials; metals and/or other like materials; alloys and/or other like materials; any other suitable material; and/or any combination thereof.

For the exemplary purposes of this disclosure, as a restatement of or in addition to what has already been described and disclosed above, the FO or PRO membranes used in various implementations may be constructed of a wide variety of materials and have a wide variety of operating characteristics. For example, the membranes may be semi-permeable, meaning that they pass substantially exclusively the components that are desired from the solution of higher concentration to the solution of lower concentration, for example, passing water from a more dilute solution to a more concentrated solution. Any of a wide variety of membrane types may be utilized using the principles disclosed in this document.

Also, as a restatement of or in addition to what has already been described and disclosed above, the FO or PRO membranes used in various implementations may be made from a thin film composite RO membrane or a membrane cast by an immersion precipitation process (which could be cast on a porous support fabric such as woven or nonwoven nylon, polyester or polypropylene). The membranes used may be hydrophilic, membranes with salt rejections in the 80% to 95% range when tested as a reverse osmosis membrane (60 psi, 500 PPM NaCl, 10% recovery, 25.degree. C.). The nominal molecular weight cut-off of the membrane may be 100 daltons. The membranes may be made from a hydrophilic membrane material, for example, cellulose acetate, cellulose proprianate, cellulose butyrate, cellulose diacetate, blends of cellulosic materials, polyurethane, polyamides. The membranes may be asymmetric (that is, for example, the membrane may have a thin rejection layer on the order of five (5) or less microns thick and a dense and porous sublayers up to 300 microns thick overall) and may be formed by an immersion precipitation process. The membranes are either unbacked, or have a very open backing that does not impede water reaching the rejection layer, or are hydrophilic and easily wick water to the membrane. Thus, for mechanical strength they may be cast upon a hydrophobic porous sheet backing, wherein the porous sheet is either woven or non-woven but having at least about 30% open area. The woven backing sheet may be a polyester screen having a total thickness of about 65 microns (polyester screen) and total asymmetric membrane is 105 microns in thickness. The asymmetric membrane may be cast by an immersion precipitation process by casting a cellulose material onto a polyester screen. The polyester screen may be 65 microns thick, 55% open area.

For the exemplary purposes of this disclosure, the brines may generally be inorganic salt. For example, a brine may be Sodium chloride=6.21 wt %; Potassium chloride=7.92 wt %, Trisodium citrate=10.41 wt %, and the like.

Various organic forward osmosis system implementations may be manufactured using conventional procedures as added to and improved upon through the procedures described here. Some components defining organic forward osmosis system implementations may be manufactured simultaneously and integrally joined with one another, while other components may be purchased pre-manufactured or manufactured separately and then assembled with the integral components.

Manufacture of these components separately or simultaneously may involve extrusion, pultrusion, vacuum forming, injection molding, blow molding, resin transfer molding, casting, forging, cold rolling, milling, drilling, reaming, turning, grinding, stamping, cutting, bending, welding, soldering, hardening, riveting, punching, plating, and/or the like. If any of the components are manufactured separately, they may then be coupled with one another in any manner, such as with adhesive, a weld, a fastener, wiring, any combination thereof, and/or the like for example, depending on, among other considerations, the particular material forming the components.

For the exemplary purposes of this disclosure, in one implementation a process for making a spiral wound membrane filter element or module may include: (a) assembling an envelope sandwich; (b) assembling a center tube onto the envelope sandwich; and (c) wrapping the envelope sandwich having the center tube and glue to form the spiral wound membrane module.

### Use

This synergistic organic FO system and process is uniquely valuable to organic food production and processing operations, and represents a significant potential advance in sustainable food process technology. The organic FO system and process simultaneously treats digester centrate, recovers digester biomass, augments that biomass with phosphorus, and recovers water for reuse.

Implementations of an organic FO system are also useful in a variety of other FO/water treatment applications, such as osmotic-driven water purification and filtration, desalination of sea water, purification of contaminated aqueous waste streams, and the like. Implementations may also be used for PRO systems. The difference is that PRO generates osmotic pressure to drive a turbine or other energy-generating device. All that would be needed is to switch to feeding fresh water (as opposed to osmotic agent) and the salt water feed can be fed to the outside instead of source water (for water treatment applications).

In places where the description above refers to particular implementations, it should be readily apparent that a number of modifications may be made without departing from the spirit thereof and that these implementations may be alternatively applied. The accompanying CLAIMS are intended to cover such modifications as would fall within the true spirit and scope of the disclosure set forth in this document. The presently disclosed implementations are, therefore, to be considered in all respects as illustrative and not restrictive, the scope of the disclosure being indicated by the appended CLAIMS rather than the foregoing DESCRIPTION. All changes that come within the meaning of and range of equivalency of the CLAIMS are intended to be embraced therein.

## Claims

1. A method for treating biomass digester centrate, comprising the steps of:
(a) subjecting the centrate to an acidic treatment to produce (i) an acid-treated centrate stream and (ii) a stream of wet solids; and
(b) subjecting the acid-treated centrate stream to forward osmosis in a forward osmosis loop, to produce (i) a concentrated acid-treated centrate stream and (ii) a diluted forward osmosis osmotic agent solution stream.

2. The method of claim 1, wherein step (a) comprises subjecting the centrate to an acidic treatment using one or more of phosphoric acid, nitric acid, citric acid, sulfuric acid, carbon dioxide, organically certified phosphoric acid, organically certified nitric acid, organically certified citric acid, and organically certified sulfuric acid.

3. The method of claim 1 or claim 2, wherein step (a) further comprises digesting the centrate prior to subjecting the centrate to acid treatment.

4. The method of claim 3, wherein methane is produced by digesting the centrate, and the method further comprises recovering the methane.

5. The method of any preceding claim, wherein step (a) further comprises screening the centrate prior to subjecting the centrate to acidic treatment.

6. The method of any preceding claim, wherein step (b) further comprises:
diverting the acid-treated centrate stream to one side of a forward osmosis membrane, contacting an opposite side of the forward osmosis membrane with a forward osmosis agent solution in a forward osmosis draw loop and osmotically pulling water across the at least one forward osmosis membrane from the acid-treated digester centrate stream to the osmosis agent solution using only a concentration gradient,
thereby producing (i) the concentrated, acid-treated centrate stream and (ii) the diluted forward osmosis osmotic agent solution stream.

7. The method of any preceding claim, further comprising recovering the stream of wet solids produced in step (a).

8. The method of any preceding claim, further comprising filtering and drying the stream of wet solids.

9. The method of any preceding claim, further comprising (c) subjecting the diluted forward osmosis osmotic agent solution stream to reverse osmosis, to produce (i) a concentrated osmotic agent solution stream and (ii) a substantially pure water stream.

10. The method of claim 9, further comprising using the concentrated osmotic agent solution stream produced in step (c) in the forward osmosis loop in step (b).

11. The method of any preceding claim, wherein step (a) further comprises subjecting the stream of wet solids to filtering, to produce a reject particle stream and a screened liquid stream.

12. The method of 11, further comprising (i) drying the reject particle stream, and (ii) subjecting the screened liquid stream to ultrafiltration or microfiltration, to produce a stream of produced water and stream of reject water.

13. The method of claim 12, further comprising returning the stream of produced water to the forward osmosis loop of step (b).

14. The method of claim 14, further comprising combining the reject water stream with the centrate.

15. The method of claim 1, comprising:
(a) subjecting the centrate to an acidic treatment to produce (i) an acid-treated centrate stream and (ii) a stream of wet solids;
(b) subjecting the acid-treated centrate stream to forward osmosis in a forward osmosis loop, to produce (i) a concentrated acid-treated centrate stream and (ii) a diluted forward osmosis osmotic agent solution stream;
(c) subjecting the diluted forward osmosis osmotic agent solution stream to reverse osmosis, to produce (i) a concentrated osmotic agent solution stream and (ii) a substantially pure water stream;
(d) returning the concentrated osmotic agent solution stream to the forward osmosis loop of step (b);
(e) filtering the stream of wet solids to produce (i) a stream containing a high concentration of wet solids and (ii) a stream containing a low concentration of wet solids;
(f) filtering the stream containing a low concentration of wet solids, to produce a reject particle stream and a screened liquid stream;
(g) subjecting the screened liquid stream to ultrafiltration or microfiltration, to produce a stream of produced water and stream of reject water;
(h) returning the stream of produced water to the forward osmosis loop of step (b);
(i) combining the stream of reject water stream with the centrate in step (a); and
(j) repeating steps (a) through (i).

16. A method for producing fertilizer from biomass digester centrate, comprising the steps of:
performing steps (a) and (b) of claim 1; and
(c) recovering the stream of wet solids for use as fertilizer.

17. The method of claim 16, comprising:
(a) subjecting the centrate to an acidic treatment to produce (i) an acid-treated centrate stream and (ii) a stream of wet solids;
(b) subjecting the acid-treated centrate stream to forward osmosis in a forward osmosis loop, to produce (i) a concentrated acid-treated centrate stream and (ii) a diluted forward osmosis osmotic agent solution stream;
(c) subjecting the diluted forward osmosis osmotic agent solution stream to reverse osmosis, to produce (i) a concentrated osmotic agent solution stream and (ii) a substantially pure water stream;
(d) returning the concentrated osmotic agent solution stream to the forward osmosis loop of step (b);
(e) filtering the stream of wet solids to produce (i) a stream containing a high concentration of wet solids and (ii) a stream containing a low concentration of wet solids;
(f) filtering the stream containing a low concentration of wet solids, to produce a reject particle stream and a screened liquid stream;
(g) subjecting the screened liquid stream to ultrafiltration or microfiltration, to produce a stream of produced water and stream of reject water;
(h) returning the stream of produced water to the forward osmosis loop of step (b);
(i) combining the stream of reject water stream with the centrate in step (a); and
(j) recovering the stream of wet solids for use as fertilizer.

18. The method of claim 16 or claim 17, wherein the step of recovering the stream of wet solids for use as fertilizer further comprises filtering the stream of wet solids to produce solids, drying the solids, and recovering the solids for use as fertilizer.

19. Apparatus comprising:
a separation tank (40) configured to subject a biomass digester centrate to an acidic treatment to produce (i) an acid-treated centrate stream and (ii) a stream of wet solids; and
a forward osmosis loop (70) configured to subject the acid-treated centrate stream to forward osmosis to produce (i) a concentrated acid-treated centrate stream and (ii) a diluted forward osmosis osmotic agent solution stream.
